# EUROPEAN PATENT SPECIFICATION

(11) **EP 1 785 089 B1**
(45) Date of publication and mention of the grant of the patent: **21.07.2010**
(21) Application number: 05806247.2
(22) Date of filing: 08.11.2005
(51) Int. Cl.: A61B 5/145, G01N 21/27

(54) **BIOINFORMATION MEASURING OPTICAL ELEMENT AND BIOINFORMATION MEASURING INSTRUMENT USING SAME**
OPTISCHES BIOINFORMATIONSMESSELEMENT UND BIOINFORMATIONSMESSINSTRUMENT DAMIT
ELEMENT OPTIQUE DE MESURE DE BIOINFORMATION ET INSTRUMENT DE MESURE DE BIOINFORMATION UTILISANT CELUI-CI

(30) Priority: 12.11.2004 JP 2004329248
(43) Date of publication of application: 16.05.2007
(73) Proprietor: Panasonic Corporation, Kadoma-shi Osaka 571-8501 (JP)
(72) Inventor: UCHIDA, Shinji c/o Matsushita Electric Indust. Co., Osaka -shi, Osaka 540-6319 (JP)
(74) Representative: TBK-Patent
(86) International application number: PCT/JP2005/020442
(87) International publication number: WO 2006/051778

(56) References cited:
- EP-A1- 1 464 273
- WO-A-03/071235
- WO-A1-01/58355
- WO-A1-03/021239
- US-A1- 2003 109 030

## Description

The present invention relates to an optical element for measuring biological information used for noninvasive measurement of glucose, cholesterol, urea, or triglycerides in a body fluid by optically measuring biological tissue, and to a biological information measuring device using the optical element.

Prior art which is related to this field of technology can be found e.g. in document US 2003/0109030 A1 disclosing a biological information collecting probe, a biological information measuring instrument, a method for producing a biological information collecting probe, and a biological information measuring method.

Conventionally, there has been proposed a method for measuring biological information by using an optical element comprising a recess portion to be brought into contact with an organism surface (for example, Patent Document 1). The biological information measuring device disclosed in Patent Document 1 comprises, as shown in FIG. 10, the light source 11; the contact element for detecting biological information having a plurality of recess portions 621, 622, 623, 624, and 625, and projecting portions 631, 632, 633, 634, 635, and 636 on the surface thereof; the light detector 16; the signalprocessing unit 64; and the display device 65.

When using this biological information measuring device, the surface of the contact element for detecting biological information is brought into contact with the surface of the biological tissue 66, and light from the light source 11 is allowed to enter into the biological tissue 66 that is in contact with the above-mentioned recess portions 621, 622, 623, 624, and 625 from the contact element for detecting biological information, while the above-mentioned projecting portions 631, 632, 633, 634, 635, and 636 are pressed into the biological tissue 66.
After propagating into the biological tissue 66, the light enters the contact element for detecting biological information, and is detected by the light detector 16 after exiting from the contact element for detecting biological information. By adjusting the depth of the recess portions 621, 622, 623, 624, and 625, the depth of the biological tissue 66 to be a target of the measurement can be controlled, and optical characteristics of a specific layer can be determined.
Patent Document 1: International Publication No. 01/58355

However, the conventional optical measuring method and optical measuring device as mentioned in the above had the following problems. In the above conventional method, for example, information at different depths is measured by forming the recess portion 622 deeper than the recess portion 623 at another position, among the plurality of recess portions 621, 622, 623, 624, and 625, and projecting portions 631, 632, 633, 634, 635, and 636. However, since a single contact element for detecting biological information was used, it was difficult to separate the light that passed through the recess portion 622 and the light that passed through the recess portion 623, and there was a possibility of interference between the both signals.
Thus, in view of the above situations, the present invention aims to provide an optical element for measuring biological information and a biological information measuring device, which can reduce interference between a plurality of signals based on lights that passed through different depths in an organism, and can measure a plurality of optical parameters related to biological information with high precision.
According to the present invention, this is accomplished by what is set forth in the appended independent claims. Advantageous modifications are defined in the appended dependent claims.

The present invention reduces the interference between a plurality of signals based on lights that passed through different depths in an organism, and can measure a plurality of optical parameters related to biological information with high precision.

[FIG. 1] A schematic diagram of an optical element for measuring biological information in embodiment 1 of the present invention, and a biological information measuring device using the optical element.
[FIG. 2] A general illustration of a structure of skin of an organism.
[FIG. 3] A schematic cross section illustrating a groove portion 18 of an optical element 13 for measuring biological information (a first groove portion 18a, a second groove portion 18b and/or a third groove portion 18c), being in close contact with a biological tissue.
[FIG. 4] A diagram illustrating a vicinity of a groove portion 18 (that is, a first groove portion 18a) when a shallow position of a biological tissue is to be measured.
[FIG. 5] A diagram illustrating a vicinity of a groove portion 18 (that is, a second groove portion 18b) when a deep position of a biological tissue is to be measured.
[FIG. 6] A schematic diagram illustrating a structure of a light separating means 14.
[FIG. 7] A schematic diagram of an optical element 13 for measuring biological information in embodiment 2 of the present invention, and of a biological information measuring device using the optical element.
[FIG. 8] An optical element 13 for measuring biological information viewed from a side where the light exits in FIG. 7 (direction of arrow X).
[FIG. 9] A schematic diagram of an optical element 13 for measuring biological information in embodiment 3 of the present invention, and of a biological information measuring device using the optical element.

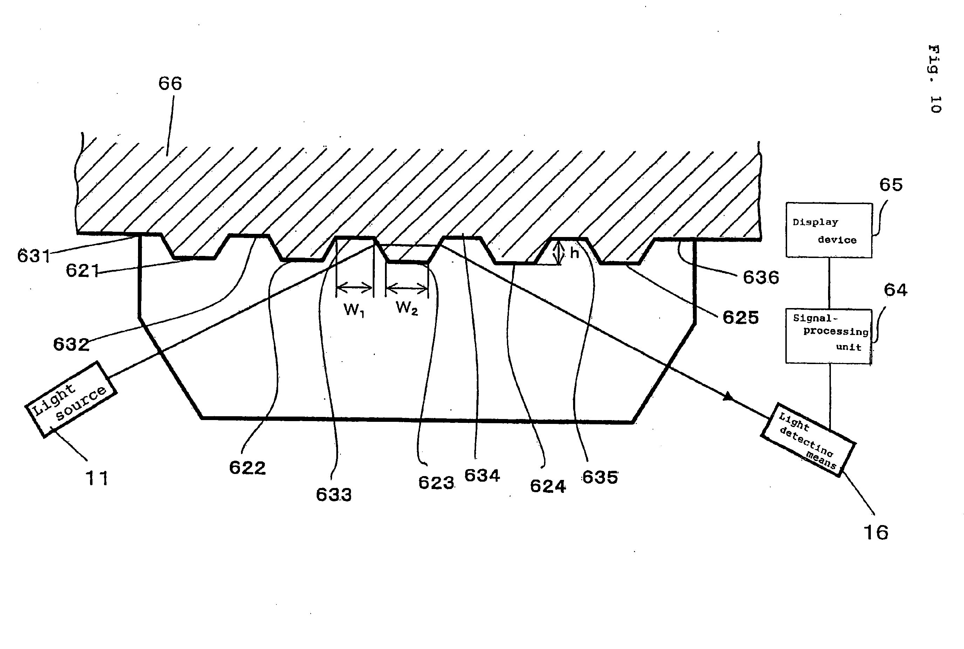

[FIG. 10] A schematic diagram illustrating a structure of a conventional biological information measuring device.

An optical element for measuring biological information of the present invention comprises a first light-transmitting body including a first groove portion to be brought into contact with an organism and to emit light to the organism and receive light, and a second light-transmitting body including a second groove portion to be brought into contact with an organism and to emit light to the organism and receive light, the second groove portion being deeper than the first groove portion.
Based on such a structure, with a simple structure comprising at least two light-transmitting bodies that are different from each other, interference between a plurality of signals based on lights that passed through different depths in an organism can be reduced, and a plurality of optical parameters related to biological information can be measured with high precision.

Between the at least two light-transmitting bodies, a light-shielding body that absorbs or reflects the light is preferably provided. Based on such a structure, since the light-shielding body prevents the light that passes through the light-transmitting body from entering adjacent light-transmitting bodies, the possibility of interference between the signal detected in the light-transmitting body and other signals is eliminated, enabling a further accurate measurement.
Also, the first light-transmitting body, the second light-transmitting body, and the light-shielding body are preferably stacked so that the light-shielding body is interposed between the first light-transmitting body and the second light-transmitting body.

In the above element for measuring biological information, a groove portion for applying light to the organism and receiving the light that passed through the tissue is preferably provided at least one of the organism contacting portions, among the organism contacting portion of the first light-transmitting body and the organism contacting portion of the second light-transmitting body.
Based on such a structure, especially on the groove portion, light can be applied to a specific portion of the biological tissue to obtain exit light, and measurement can be carried out for that specific portion.

The optical element for measuring biological information in the present invention may further comprise a light-transmitting body (third light-transmitting body) for measurement of a surface of an organism.
Based on such a structure, homogeneity of the organism and surface conditions of the optical element can be evaluated further in detail, enabling detection of measurement errors and achieving measurement with further precision.

The present invention also provides a biological information measuring device comprising a light source; the optical element for measuring biological information mentioned above; a light detector for detecting light exited the optical element; and a computing unit for arithmetically processing information obtained by the light detector to calculate biological information.
Based on the biological information measuring device of the present invention, a concentration of a target component in the biological tissue can be measured reliably and noninvasively.

In the following, an embodiment of the optical element for measuring biological information and the biological information measuring device in the present invention is described in detail by referring to the drawings. However, the following embodiments are examples, and the present invention are not limited to these examples.
In the description below, same reference numerals are used for the same or corresponding part, and repetitive descriptions may be omitted.

### <<Embodiment 1>>

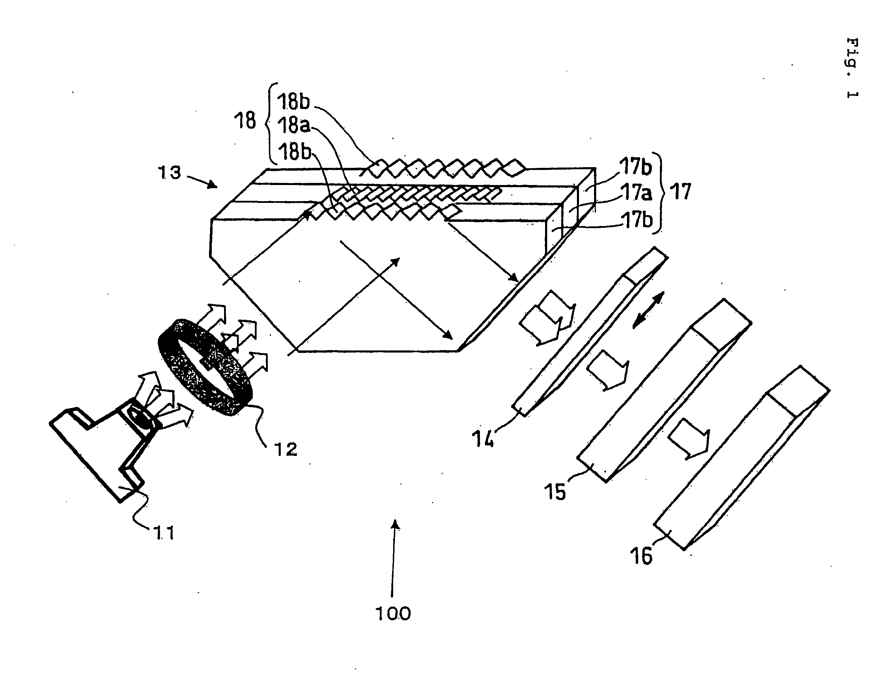

FIG. 1 is a schematic diagram of an optical element for measuring biological information in an embodiment of the present invention, and a biological information measuring device using the optical element. As shown in FIG. 1, a biological information measuring device 100 in this embodiment is formed with a light source 11, an optical lens 12, an optical element 13 for measuring biological information, a light separating means 14, a spectroscopic means 15, and a light detecting means 16.

The positional relationship between the light separating means 14 and the spectroscopic means 15 is not limited to the position shown in FIG. 1. The light separating means 14 and the spectroscopic means 15 can be inserted between the light source 11 and the optical element 13 for measuring biological information. The spectroscopic function can also be carried out by changing the wavelength of the light source 11, without using the spectroscopic means 15 and the light separating means 14.

For the light source 11, a light source that can emit light including a light component of a wavelength that can be absorbed by the measurement target (substance) in the biological tissue will suffice. For the light source emitting light of the mid-infrared range, for example, a Globar light source which is SiC sintered into a rod, a CO₂ laser, a tungsten lamp, an infrared pulse light source, and a quantum cascade laser (QCL) light source may be used.

When a substance having a strong absorption peak in the mid-infrared region such as in the proximity of wave numbers of 1033 cm⁻¹ and 1080 cm⁻¹, such as glucose, without particular limitation, a Globar light source, an infrared pulse light source, and a QCL light source are preferable. When a substance having an absorption in the near-infrared range is to be measured, for example, a halogen light source, a semiconductor laser, and an LED are preferably used. Glucose is known to have an absorption peak in the near-infrared range in addition to the mid-infrared range: therefore, especially an LED is preferably used.

For the material forming the optical lens 12 and the light-transmitting body 17, known ones in the art may be used. For example, when a substance having an absorption in the mid-infrared range is to be measured, silicon, germanium, SiC, diamond, ZnSe, ZnS, and KrS may be used.
When a substance having an absorption peak in the mid-infrared range, i.e., a wave number of 1033 cm⁻¹ or 1080 cm⁻¹, such as glucose is to be measured, silicon or germanium is particularly preferable, in view of high transmission of an infrared wavelength of about 9 to 10 microns, and also workability and high mechanical strength. When a substance having an absorption in the near-infrared range, fused quartz, single crystal silicon, optical glass, and plastic are preferably used.

The optical element 13 for measuring biological information in this embodiment comprise a first light-transmitting body 17a and two second light-transmitting bodies 17b disposed to sandwich the first light-transmitting body. The first light-transmitting body 17a and the second light-transmitting body 17b comprises a first groove portion 18a and a second groove portion 18b, respectively. The second groove portion 18b is formed deeper than the first groove portion 18a.
With regard to the shape of the first groove portion 18a and the second groove portion 18b, in view of ease in its production, for example, V-shaped grooves as shown in FIG. 1 are preferably used. The first groove portion 18a and the second groove portion b are not limited to the V-shape. For example, the shape may be U-shape, recessed, or staircase-like. Although the first groove portion 18a and the second groove portion 18b have the same shape in FIG. 1, the shape may be different.

The first groove portion 18a and the second groove portion 18b may be formed by using known production technique.
For example, when a crystal of silicon or germanium is to be used as a material forming the first light-transmitting body 17a and the second light-transmitting body 18b, the first groove portion 18a and the second groove portion 18b may be formed by using an anisotropic etching technique.
When resin is used as a material forming the first light-transmitting body 17a and the second light-transmitting body 18b, the first groove portion 18a and the second groove portion 18b may be formed simultaneously with the formation of the first light-transmitting body 17a and the second light-transmitting body 18b by using a molding method.

The first light-transmitting body 17a in FIG. 1 comprises a first groove portion 18a that is shallower than that of the two second light-transmitting bodies 17b, and can be used for measuring the surface of the organism. That is, homogeneity of the organism and the surface conditions of the optical element are evaluated further in detail with the first light-transmitting body 17a, and measurement with a further precision can be carried out by detecting measurement error.

Generally, accurate measurement may be difficult when sweat, soil, saliva, and the like are present in a large quantity between the organism and the measurement part, and also when the amount and quality of these change at every measurement time.
Thus, in this embodiment, second groove portions 18b are preferably provided. Presence of sweat, soil, and saliva, that are present in many cases at a skin surface, in the range of about several tens of micrometer, can thus be grasped. The depth of the groove in the second groove portion 18b is preferably several tens of micrometers or more. By using such grooves, soil on the skin, contact on the skin, and homogeneity of skin surface conditions can be easily detected. By using the detection result to detect an error, highly precise measurement can be achieved.

That is, an abnormality can be determined by measuring the spectrum of returning light from the second groove portion 18b, calculating the absorbance of a specific wavelength, and determining deviation from the standard value. For example, when sweat and saliva are present in quantity on the surface layer, it can be determined since the absorbance will be almost the same with water.
When the detection result showed a similar absorbance with water, it is preferable to stop the measurement, and wipe off the soil such as water from the skin surface, because an accurate measurement is difficult.

The abnormality in the contact with the skin and homogeneity in skin surface conditions is preferably detected by providing two second light-transmitting bodies 17b to provide two second groove portions 18b.
That is, when the signals from the two second groove portions 18b are compared and found to be greatly different, surface conditions of the skin may not be homogenous, or there may be a problem in the contacting conditions, and it is preferable to stop the measurement and wipe off the soil on the biological tissue surface. Also, even when the signals from the two second groove portions 18b are equal, if the signal shows that excessive soil on the surface layer is included, it is preferable to stop the measurement and wipe off the soil on the skin surface. The soil includes oil, other than water.

When the signals from the two second groove portions 18b are appropriate and almost equal, it is determined that the conditions of the organism surface are homogenous, and that contacting conditions are appropriate. Thus, when such determination is made, the measurement is started, and biological information is measured by the first groove portion 18a of the first light-transmitting body 17a disposed between the two second light-transmitting bodies 17b.
By thus evaluating the surface conditions of the optical element and its homogeneity with the two second groove portions 18b, further accurate measurement is achieved compared with the case in which only the light-transmitting body is used.

A space may be provided between the first light-transmitting body 17a and the second light-transmitting body 17b, for example, by interposing a spacer. Based on such a structure, light is reflected by the difference in the refractive index of air existing in the space, and the refractive index of the light-transmitting body, generating light-shielding effects.

For the light separating means 14, a known one in the art may be used without any particular limitation, as long as it separates the light that measured the respective depths, and allows the light to enter the spectroscopic means 15 and the light detecting means 16. For example, a mechanical chopper, a filter, and an acousto-optic modulator may be used.
The spectroscopic means 15, though not shown in FIG. 1 in detail, is not particularly limited, and known spectroscopic technique may be used, including diffraction spectroscopic method using a grating, a method using an interference filter, and an FT-IR method.

For the light detecting means 16, known ones in the art may be used. For example, for the mid-infrared range, a pyroelectric sensor, a thermopile, a thermistor, and an MCT detector (HgCdTe detector, a kind of quantum detector) may be mentioned. For the near-infrared range, an InGaAs detector, a photodiode, a PbS detector, an InSb detector, and an InAs detector may be mentioned.
Though not shown, by carrying out calculation with a computing means such as a computer based on the signal detected by the light detecting means 16, for example, parameters of the biological tissue such as a glucose concentration can be calculated.

An operation of the biological information measuring device 100 in this embodiment is described by using FIG. 1.
As shown in FIG. 1, light exited from the light source 11 and entered the optical lens 12 reaches the optical element 13 for measuring biological information. A portion of the reached light enters the light-transmitting body 17 (the first light-transmitting body 17a, the second light-transmitting body 17b, and/or the third light-transmitting body 17c), and reaches the groove portion 18 (the first groove portion 18a, the second groove portion 18b, and/or the third groove portion 18c) formed on the light-transmitting body 17.

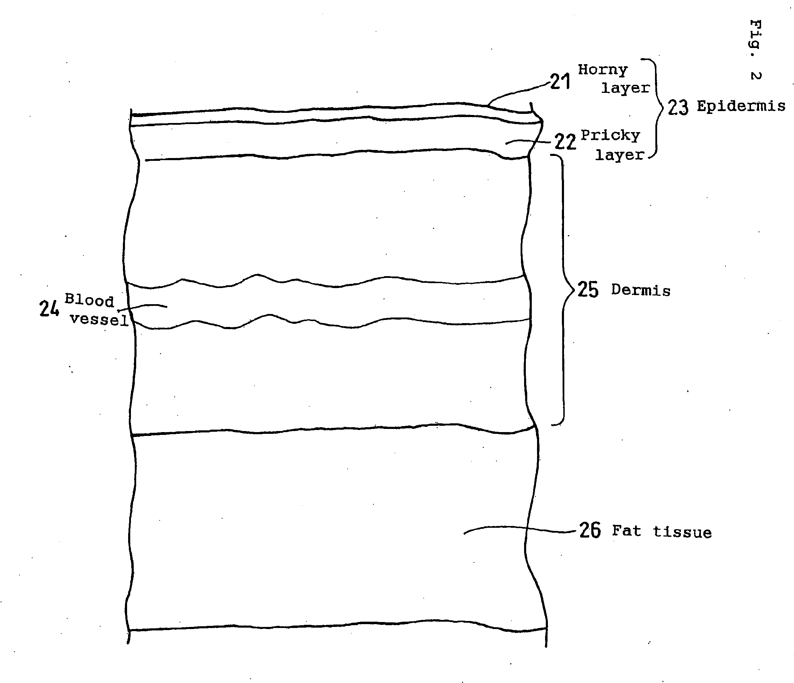

FIG. 2 is a general illustration of a structure of a skin of an organism. The outermost layer is called a horny layer 21, the tissue where almost no glucose exist. A prickly layer 22 is a tissue where glucose penetrated from a blood vessel 24 in dermis 25 exists comparatively in large amount. The horny layer 21 and the prickly layer 22 together are generally called epidermis. The tissue below the epidermis 23 is called dermis 25, and is a region where there are many blood vessels 24 and the glucose concentration is high. Below the dermis 25 is called fat tissue 26, where a glucose concentration is generally low compared with the dermis 25.
The thickness of the horny layer 21 varies depending on the part of the organism. For example, in the case of cheeks, the thickness of the horny layer 21 is 0.01 to 0.02 mm, the thickness of the prickly layer 22 is 0.08 to 0.28 mm, and the thickness of the dermis 25 is 2 to 3 mm.

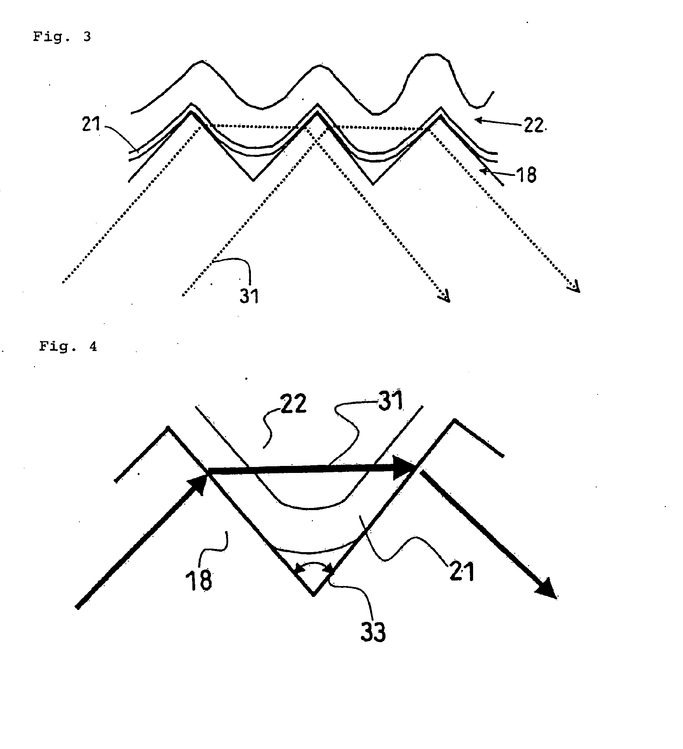

FIG. 3 is a schematic cross section illustrating a groove portion 18 of an optical element 13 for measuring biological information, being in close contact with a biological tissue. As shown in FIG. 3, when an organism having a tissue such as the horny layer 21 and the prickly layer 22 is pressed against the groove portion 18 to bring it into close contact, the organism bore the form as shown in FIG. 4, and thus transmission characteristics of a specific portion of the tissue can be measured easily.
That is, when light 31 is allowed to enter the organism through the groove portion 18, the light 31 is refracted at an interface between the horny layer 21 and the groove portion 18, and enters the horny layer 21 at a certain angle. The light being allowed to enter travels in straight lines in the organism, and after penetrating the prickly layer 22, the light 31 is refracted at an interface between the horny layer 21 and the groove portion 18 again, to reach the groove portion 18.

To refract the light 31 at the interface between the groove portion 18 and the horny layer 21 as shown in FIG. 4, the refractive index of the light-transmitting body 17 provided with the groove portion 18 is preferably higher than the horny layer 21 and the prickly layer 22.
Although the shape and the size of the groove portion 18 are not particularly limited, for example, the V-shaped groove portion as shown in the above may be used.

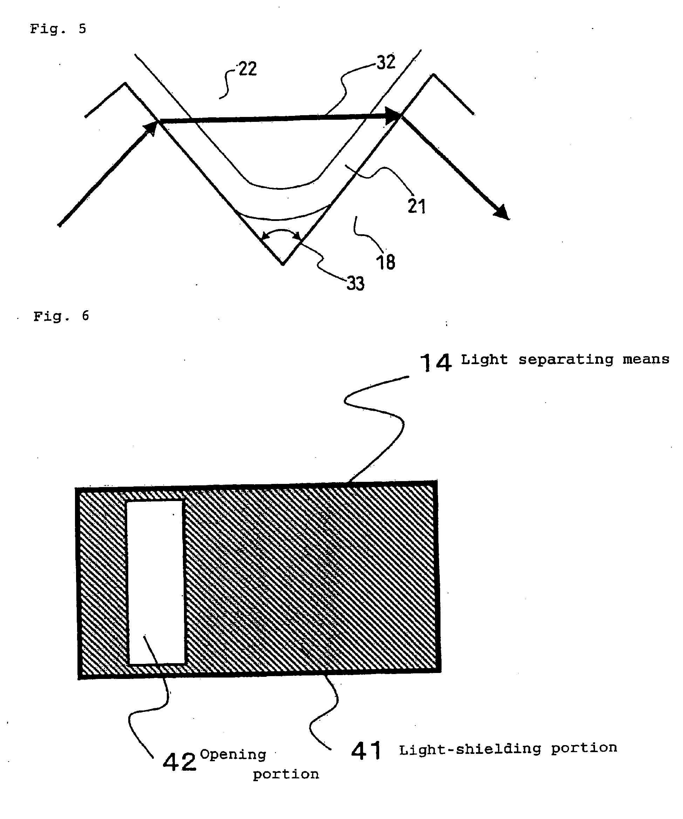

FIGs. 4 and 5 are a partially enlarged view of the groove portion 18 in FIG. 3. To be specific, FIGs. 4 and 5 are diagrams showing a method for measuring a different depth in an organism, by changing for example a depth of the groove portion 18.
FIG. 4 is a diagram illustrating a vicinity of the groove portion 18 (that is, a first groove portion 18a) when a shallow position of the biological tissue is to be measured. FIG. 5 is a diagram illustrating a case when a deep position is to be measured, and an organism having a tissue comprising the horny layer 21 and the prickly layer 22 is contacting the groove portion 18 (that is, a second groove portion 18b).

For light used for the measurement, for example, light in the near-infrared range with a wavelength of 1.2 µm or more and 2.5 µm or less, and in the mid-infrared range with a wavelength of 2.5 to 10 µm may be used.
Among these, the mid-infrared range is preferable in that an absorption inherent to a substance exists in the range and any component can be easily identified. Since light in the mid-infrared range is largely absorbed by an organism, the depth of penetration into an organism is about 200 µm. On the other hand, light in the near-infrared range is less absorbed by the organism, and measurement is possible even at a depth of 200 µm or more.

For the biological tissue measured by the optical element for measuring biological information and the biological information measuring device in the present invention, those parts where epidermis is present, such as an inner side of a forearm, earlobe, lip mucosa, finger, and upper arm of humans may be mentioned.
For example, lips do not include the horny layer and the dermis, and the epidermis layer has a thickness of 50 to 200 µm. Thus, when an organism component in the epidermis layer of the lips, for example a concentration of glucose, is to be measured, light in the mid-infrared range may be used. At this time, both of the shallow groove portion and the deep groove portion can be set to a depth of 200 µm or less.
Based on such, by deducting the transmission data obtained by the shallow groove from the transmission data obtained by the deep groove (that is, measurement data obtained from the transmitted light), effects from sweat, soil, and saliva present in organism surface may be restrained.

For the material of the light-transmitting body 17, for example, silicon that is transparent in the mid-infrared range is used. When silicon is used, by applying an anisotropic etching process, a known techniques in silicon crystal, the V-shape groove may be achieved easily.
For example, any of a vertex angle 33 of the groove portion 18 is processed to give 70.6°. Also, by selecting crystal processed surface of silicon, the vertex angle 33 may be processed to give 117°.
The present invention does not particularly limit the vertex angle 33 of the groove portion 18, but depending on the thickness and hardness of the epidermis of the biological tissue to be measured, it may be selected appropriately.

Also, when the measurement is to be carried out at a finger cushion, the thickness of the horny layer is about 200 µm, the thickness of the epidermis layer is about 240 µm, and the thickness of the total of the horny layer, the epidermis layer, and the dermis layer is about 2 mm. Thus, when the depths of both the shallow groove portion and the deep groove portion are set to 440 µm to 2 mm, by deducting the transmission data obtained by the shallow grooves from the transmission data obtained by the deep grooves, a component of an organism in the dermis layer can be measured, removing the effects from the horny layer and the epidermis layer.
Especially when the component of the organism is glucose, it is preferable since dermis includes glucose in quantity. For the light to be used for the measurement at this time, it is preferable to use the light in the near-infrared range with less absorption by the organism.
Silicon may be used for the material of the light-transmitting body 17, as in the case of the mid-infrared range, but it is not limited thereto and glass and resin may be used as well.

Thus, a relatively shallow portion of the biological tissue can be measured by the groove portion 18 (a shallow first groove portion 18a) in FIG. 4, and a deeper portion can be measured by the groove portion 18 (the second groove portion 18b more deeper than the first groove portion 18a) in FIG. 5.
Further, as shown in for example FIG. 1, by stacking a plurality of light-transmitting bodies 17 having the groove portion of different depths, the measurement can be carried out for variety of depths.

As shown in FIGs. 3 to 5, light that passes through the biological tissue via the groove portion 18 and returned again exits the light-transmitting body 17 and then reaches the light separating means 14, as shown in FIG. 1.
FIG. 6 is a schematic diagram showing a structure of the light separating means 14. The light exited the light-transmitting body 17 reaches the light separating means 14. The light separating means 14 shown in FIG. 6 comprises a light-shielding portion 41 and an opening portion 42. The light separating means 14 is used to separate the light exited the light-transmitting body 17 individually for the measurement, as shown also in FIG. 1.

That is, by setting the light separating means 14 so that the opening portion 42 is aligned with any of the plurality of light-transmitting bodies 17, only the light exited the light-transmitting body 17 can be allowed to pass through. Thus, by separating the organism signal from the groove portion 18 provided in the light-transmitting body 17 and the signal from the other groove portion 10 provided in the light-transmitting body 17, the detection can be carried out with high sensitivity individually.
Additionally, although the spectroscopic means 15 shown in FIG. 1 is not illustrated in detail, it separates the light that passes through the light separating means 14 to some light components each having different wavelengths.

The width of the opening portion 42 of the light separating means 14 is preferably the width that can receive the light exited the light-transmitting body 17 provided at the biological information measuring device 100. Without particular limitation, similarly with the thickness of the light-transmitting body 17 of the biological information measuring device 100, the same width is preferable.
The position of the light separating means 14 at the time of the measurement is preferably decided in correspondence to the light-transmitting body 17.
Although not shown, the light separating means 14 is preferably moved in correspondence to for example the transmitting body 17 as shown in FIG. 1, to detect the light exited the respective the transmitting bodies 17.

The light spectroscopically analyzed with the spectroscopic means 15 enters the light detecting means 16, and the absorbance is calculated based on the intensity of light.
Also, although not shown, the obtained absorbance is used to calculate by using the computing means, to calculate a glucose concentration in the body fluid. In the present invention, since the measurement can be carried out for both the information on the shallow surface layer tissue and on the deeper tissue, an accurate glucose concentration can be calculated.

### «Embodiment 2»

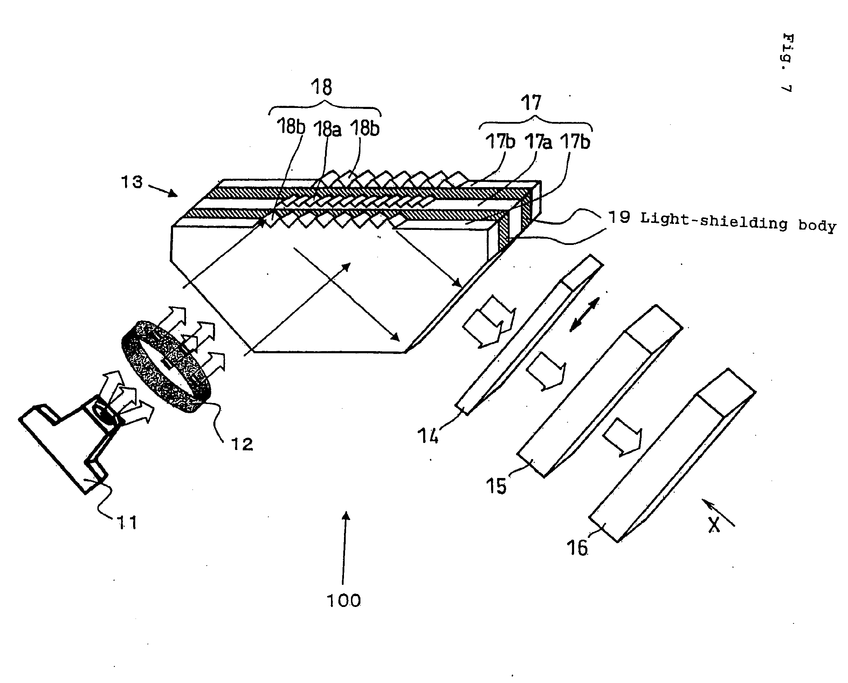

Light passing the first light-transmitting body 17a, the second light-transmitting body 17b, and the third light-transmitting body 17c in embodiment 1 as shown in FIG. 1 may partially interfere with one another. In view of preventing the interference further reliably, the optical element for measuring biological information in this embodiment is formed as illustrated in FIG. 7.
FIG. 7 is a schematic diagram of an optical element 13 for measuring biological information in this embodiment, and a biological information measuring device using the optical element. In FIG. 7, the light-shielding body 19 is disposed between the first light-transmitting body 17a and the two second light-transmitting bodies 17b. Based on such a structure, the light-shielding body 19 prevents the signals from the first light-transmitting body 17a and the second light-transmitting body 17b from interfering each other and is extremely preferable.

The light-shielding body 19 is preferably provided at a portion where the light of the first light-transmitting body 17a and the second light-transmitting body 17b do not transmit. For example, when any of the first light-transmitting body 17a and the second light-transmitting body 17b is included in a plural number, the light-shielding body 19 is preferably provided between adjacent light-transmitting bodies. Therefore, the optical element for measuring biological information in the present invention may include a plurality of light-shielding bodies 19.
For the material forming the light-shielding body 19, a material that absorbs or reflects light may be used. For example, metals that can shield the light to be used, such as aluminum, tungsten, molybdenum, chromium, and copper are preferable. Further, a thin film of these metals, or a multilayer film of these metal films and dielectric films may be preferably formed on a substrate by vapor deposition and sputtering for the usage. Also, metal foils such as aluminum foil may be used as the light-shielding body 19, and the metal foil may be sandwiched by the light-transmitting body and joined. The light-shielding body 19 may be a plate-like.

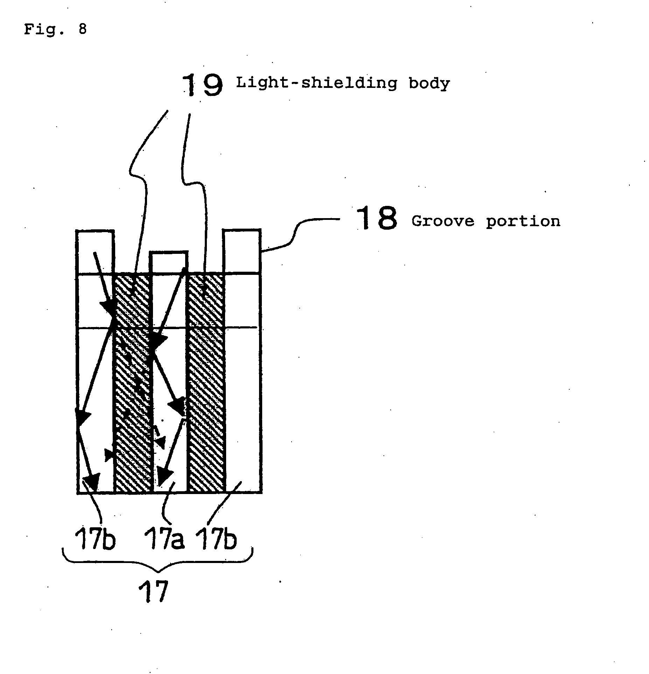

The advantages of the above light-shielding body 19 are described in further detail with reference to FIG. 8. FIG. 8 is a diagram of an optical element 13 for measuring biological information seeing from the side where light exits in FIG. 7 (the direction of arrow X).
The first light-transmitting body 17a, the two second light-transmitting bodies 17b and the two light-shielding bodies 19 are stacked. On the part of the first light-transmitting body 17a and the second light-transmitting body 17b where the organism is brought into contact, the first groove portion 18a and the second groove portion 18b are formed, respectively.

Based on such a structure, as can be seen in FIG. 8, the light passing through the first light-transmitting body 17a and the second light-transmitting body 17b partially reaches the boundary of the light-shielding body 19 without traveling straight in the center part of the first light-transmitting body 17a and the second light-transmitting body 17b.
Such light can be eliminated when the light exited the light source 11 is made a parallel beam completely, and when allowed to enter the first light-transmitting body 17a and the second light-transmitting body 17b absolutely straight. However, the light source itself is a luminous body with a diameter of about 1 mm or more in many cases, it is difficult to make a complete parallel beam, and to make such ideal conditions.

On the other hand, in this embodiment, when incident angle of the light reaching the boundary of the light-shielding body 19 is smaller than the angle of the total reflection, light exits the first light-transmitting body 17a and the second light-transmitting body 17b and reaches the light-shielding body 19.
When there is no light-shielding body 19, light re-enters the adjacent light-transmitting body, and interferes with the signal detected in the light-transmitting body, and as in the present invention, when the light-shielding body 19 that can sufficiently shield light is provided in the optical element 13 for measuring biological information, since light is mostly absorbed by or reflected at the light-shielding body 19, light does not enter the adjacent light-transmitting body 17. Therefore, light exited the light-transmitting body 17 and reflected at the light-shielding body 19 can return to the same light-transmitting body 17, increasing the amount of signal and is preferable.

Although a plate-like light-shielding body is described as a form of the light-shielding body 19, it is not limited thereto.
A metal film may be coated on the light-transmitting body surface by vapor deposition and sputtering, or a metal foil such as aluminum foil may be sandwiched between the light-transmitting bodies and joined. An adhesive layer may be provided for the joining.
Other structures may be the same with the above embodiment 1.

### «Embodiment 3»

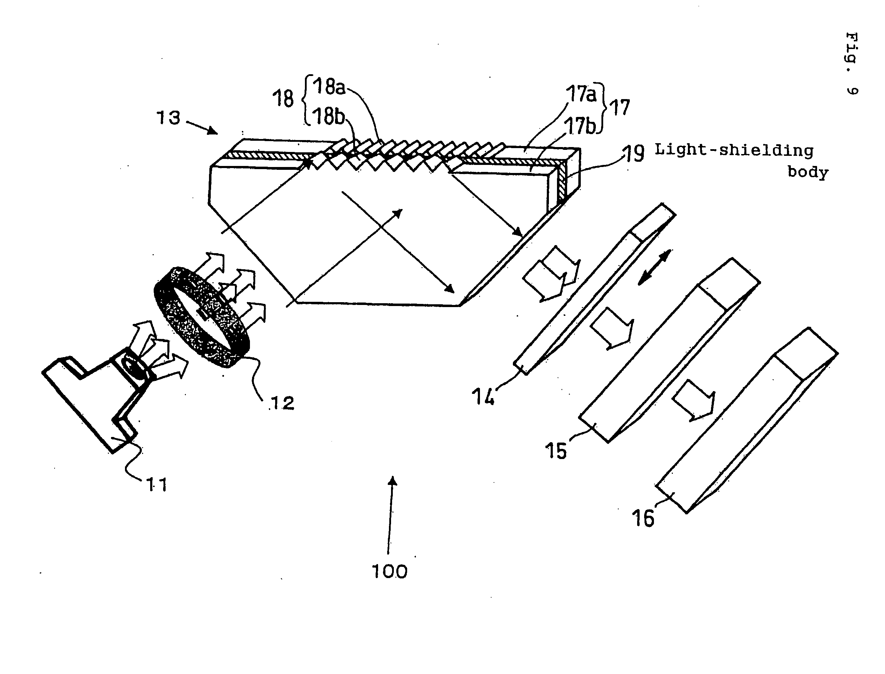

As a further simple structure of the optical element for measuring biological information in the present invention, the one shown in FIG. 9 may be mentioned. FIG. 9 is a schematic diagram of an optical element 13 for measuring biological information in this embodiment, and a biological information measuring device using the optical element.
In FIG. 9, a light-shielding body 19 is disposed between the first light-transmitting body 17a and the second light-transmitting body 17b. Based on such a structure, the light-shielding body 19 prevents interference of signals from the first light-transmitting body 17a and the second light-transmitting body 17b and is extremely preferable.
Other structures may be the same with the above Embodiment 1 or Embodiment 2.

The biological tissue measured by the optical element for measuring biological information and the biological information measuring device in the present invention is not particularly limited, and where epidermis is present, such as inner side of frontal arm, earlobe, lip mucosa, finger, and upper arm of humans, will suffice.
The measurement target may be a substance that is optically measurable such as a body fluid, and a body fluid component other than glucose can also be measured.

The optical element for measuring biological information and the biological information measuring device in the present invention can carry out accurate measurement on the surface layer tissue and a layer deeper than the surface layer selectively, and can carry out accurate measurement of information on the body fluid components. Additionally, not only the biological tissue, but various liquid samples can be measured as well, and the present invention is especially useful for the measurement of body fluid components for medical purposes.

## Claims

1. An optical element for measuring biological information, in which information of an organism is measured by applying light to said organism and using light returned from a biological tissue of said organism, the optical element comprising:
a first light-transmitting plate (17a) comprising a first groove portion (18a) which is to be brought into contact with said organism and which applies light to said organism and receives light; and
a second light-transmitting plate (17b) comprising a second groove portion (18b) which is to be brought into contact with said organism and which applies light to said organism and receives light, wherein
said first light-transmitting plate (17a) and said second light-transmitting plate (17b) are stacked, and
said second groove portion (18b) being deeper than said first groove portion (18a).

2. The optical element for measuring biological information in accordance with claim 1, wherein a light-shielding body (19) for absorbing or reflecting said light is provided between said first light-transmitting plate (17a) and said second light-transmitting plate (17b).

3. A method for measuring biological information, using an optical element for measuring biological information comprising: a first light-transmitting plate (17a) comprising a first groove portion (18a) which is to be brought into contact with an organism and which applies light to said organism and receives light; and a second light-transmitting plate (17b) comprising a second groove portion (18b) which is to be brought into contact with said organism and which applies light to said organism and receives light, wherein
said first light-transmitting plate (17a) and said second light transmitting plate (17b) are stacked, and
said second groove portion (18b) being deeper than said first groove portion (18a);
the method comprising the steps of:
applying light to an organism contacting said optical element for measuring biological information;
detecting an intensity of light returned from said organism to said first groove portion (18a);
detecting an intensity of light returned from said organism to said second groove portion (18b); and
obtaining biological information in a dermis layer of said organism, based on a difference of the detected intensity of light returned to said second groove portion (18b) and intensity of light returned to said first groove portion (18a).

4. A biological information measuring device comprising:
a light source (11); the optical element for measuring biological information in accordance with claim 1 or 2; a light detector (16) for detecting light that exited said optical element; and a computing unit for calculating biological information by arithmetically processing information obtained by said light detector (16).

## Patentansprüche

1. Optisches Element zur Messung biologischer Informationen, bei dem Informationen über einen Organismus durch Applizieren von Licht an diesen Organismus und durch Verwenden des von einem biologischen Gewebe dieses Organismus zurückgekehrten Lichts gemessen werden, wobei das optische Element umfasst:
eine erste lichtdurchlässige Platte (17a), die einen ersten Einkerbungsabschnitt (18a) umfasst, der in Kontakt mit dem Organismus zu bringen ist und welcher Licht an den Organismus appliziert und Licht empfängt; und
eine zweite lichtdurchlässige Platte (17b), die einen zweiten Einkerbungsabschnitt (18b) umfasst, der in Kontakt mit dem Organismus zu bringen ist und welcher Licht an den Organismus appliziert und Licht empfängt; wobei
die erste lichtdurchlässige Platte (17a) und die zweite lichtdurchlässige Platte (17b) gestapelt sind und
der zweite Einkerbungsabschnitt (18b) tiefer als der erste Einkerbungsabschnitt (18a) ist.

2. Optisches Element zur Messung biologischer Informationen nach Anspruch 1, wobei zwischen der ersten lichtdurchlässigen Platte (17a) und der zweiten lichtdurchlässigen Platte (17b) ein Licht abschirmender Körper (19) zum Absorbieren oder Reflektieren des Lichts bereitgestellt ist.

3. Verfahren zum Messen biologischer Informationen unter Verwendung eines optischen Elements zur Messung biologischer Informationen, wobei das Element umfasst:
eine erste lichtdurchlässige Platte (17a), die einen ersten Einkerbungsabschnitt (18a) umfasst, der in Kontakt mit einem Organismus zu bringen ist und welcher Licht an den Organismus appliziert und Licht empfängt; und eine zweite lichtdurchlässige Platte (17b), die einen zweiten Einkerbungsabschnitt (18b) umfasst, der in Kontakt mit dem Organismus zu bringen ist und welcher Licht an den Organismus appliziert und Licht empfängt; wobei
die erste lichtdurchlässige Platte (17a) und die zweite lichtdurchlässige Platte (17b) gestapelt sind und
der zweite Einkerbungsabschnitt (18b) tiefer als der erste Einkerbungsabschnitt (18a) ist;
wobei das Verfahren die Schritte umfasst:
Applizieren von Licht an einen sich in Berührung mit dem optischen Element befindlichen Organismus zur Messung biologischer Informationen;
Detektieren einer Intensität von Licht, das von dem Organismus zu dem ersten Einkerbungsabschnitt (18a) zurückgekehrt ist;
Detektieren einer Intensität von Licht, das von dem Organismus zu dem zweiten Einkerbungsabschnitt (18b) zurückgekehrt ist; und
Beschaffen biologischer Informationen in einer Hautschicht des Organismus basierend auf einer Differenz zwischen der detektierten Intensität von zu dem zweiten Einkerbungsabschnitt (18b) zurückgekehrtem Licht und von zu dem ersten Einkerbungsabschnitt (18a) zurückgekehrtem Licht.

4. Vorrichtung zur Messung biologischer Informationen, die umfasst:
eine Lichtquelle (11); das optische Element zur Messung biologischer Informationen nach Anspruch 1 oder 2; einen Lichtdetektor (16) zur Detektion von Licht, das das optische Element verlassen hat; und eine Recheneinheit zur Berechnung biologischer Informationen durch arithmetisches Verarbeiten von Informationen, die von dem Lichtdetektor (16) beschaffen wurden.

## Revendications

1. Élément optique pour mesurer une information biologique, dans lequel une information d'un organisme est mesurée en appliquant une lumière audit organisme et en utilisant une lumière renvoyée d'un tissu biologique dudit organisme, l'élément optique comprenant :
une première plaque transmettant une lumière (17a) comprenant une première partie de rainures (18a) qui est destinée à être mise en contact avec ledit organisme et qui applique une lumière audit organisme et reçoit une lumière ; et
une deuxième plaque transmettant une lumière (17b) comprenant une deuxième partie de rainures (18b) qui est destinée à être mise en contact avec ledit organisme et qui applique une lumière audit organisme et reçoit une lumière, dans lequel
ladite première plaque transmettant une lumière (17a) et ladite deuxième plaque transmettant une lumière (17b) sont empilées, et
ladite deuxième partie de rainures (18b) étant plus profonde que ladite première partie de rainures (18a).

2. Élément optique pour mesurer une information biologique selon la revendication 1, dans lequel un corps de masquage de lumière (19) pour absorber ou réfléchir ladite lumière est prévu entre ladite première plaque transmettant une lumière (17a) et ladite deuxième plaque transmettant une lumière (17b).

3. Procédé de mesure d'une information biologique, utilisant un élément optique pour mesurer une information biologique comprenant : une première plaque transmettant une lumière (17a) comprenant une première partie de rainures (18a) qui est destinée à être mise en contact avec ledit organisme et qui applique une lumière audit organisme et reçoit une lumière ; et une deuxième plaque transmettant une lumière (17b) comprenant une deuxième partie de rainures (18b) qui est destinée à être mise en contact avec ledit organisme et qui applique une lumière audit organisme et reçoit une lumière, dans lequel
ladite première plaque transmettant une lumière (17a) et ladite deuxième plaque transmettant une lumière (17b) sont empilées, et
ladite deuxième partie de rainures (18b) étant plus profonde que ladite première partie de rainures (18a) ;
le procédé comprenant les étapes de :
application d'une lumière à un organisme au contact dudit élément optique pour mesurer une information biologique ;
détection d'une intensité de lumière renvoyée dudit organisme jusqu'à ladite première partie de rainures (18a) ;
détection d'une intensité de lumière renvoyée dudit organisme jusqu'à ladite deuxième partie de rainures (18b) ; et
obtention d'une information biologique dans une couche dermique dudit organisme, sur la base d'une différence de l'intensité détectée de lumière renvoyée jusqu'à ladite deuxième partie de rainures (18b) et de l'intensité de lumière renvoyée jusqu'à ladite première partie de rainures (18a).

4. Dispositif de mesure d'une information biologique comprenant :
une source de lumière (11) ; l'élément optique pour mesurer une information biologique selon la revendication 1 ou 2 ; un détecteur de lumière (16) pour détecter une lumière qui est sortie dudit élément optique ; et une unité de calcul pour calculer une information biologique par traitement arithmétique de l'information obtenue par ledit détecteur de lumière (16).
